# EUROPÄISCHE PATENTSCHRIFT

(11) **EP 0 983 274 B1**
(45) Veröffentlichungstag und Bekanntmachung des Hinweises auf die Patenterteilung: **23.07.2003**
(21) Anmeldenummer: 98928259.5
(22) Anmeldetag: 13.05.1998
(51) Int. Cl.: C07D 487/22, C10L 1/22, C10L 1/28, C10L 1/30, C07D 519/00, C09B 47/04

(54) **PHTHALOCYANINE UND IHRE VERWENDUNG ALS MARKIERUNGSMITTEL**
PHTHALOCYANINE AND USE OF PHTHALOCYANINE AS A MARKING AGENT
PHTALOCYANINE ET SON UTILISATION COMME AGENT DE MARQUAGE

(30) Priorität: 22.05.1997 DE 19721399
(43) Veröffentlichungstag der Anmeldung: 08.03.2000
(73) Patentinhaber: BASF AKTIENGESELLSCHAFT, 67056 Ludwigshafen (DE)
(72) Erfinder: MEYER, Frank, D-68165 Mannheim (DE); VAMVAKARIS, Christos, D-67169 Kallstadt (DE); BECK, Karin, Heidrun, D-67067 Ludwigshafen (DE); WAGENBLAST, Gerhard, D-67157 Wachenheim (DE); ALBERT, Bernhard, D-67157 Wachenheim (DE)
(86) Internationale Anmeldenummer: EP9802824
(87) Internationale Veröffentlichungsnummer: WO98052950

(56) Entgegenhaltungen:
- EP-A- 0 190 444
- WO-A-96/10620
- S.A. MIKHALEKO ET AL.: "Phthalocyanines and related compounds. XIX. Tetra- and octaamino-substituted phthalocyanines" JOURNAL OF GENERAL CHEMISTRY USSR., Bd. 51, Nr. 7, 1981, Seiten 1405-1410, XP002078329 NEW YORK US in der Anmeldung erwähnt

## Beschreibung

Die vorliegende Erfindung betrifft die Verwendung von Phthalocyaninen der Formel I 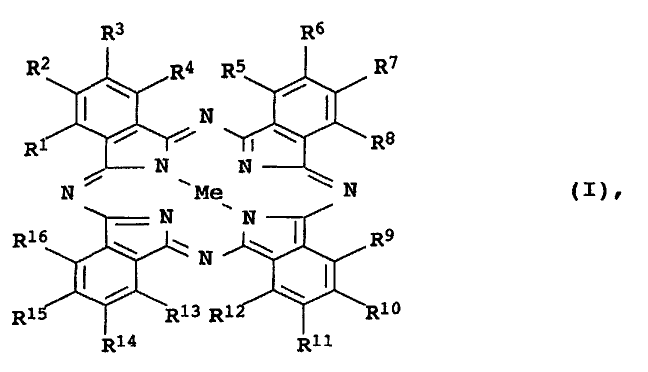 in der
Me zweimal Wasserstoff, zweimal Lithium, Magnesium, Zink, Kupfer, Nickel, VO, TiO, AlCl, AlOH, AlOCOCH₃, AlOCOCF₃, SiCl₂ oder Si(OH)₂,
mindestens vier der Reste R¹ bis R¹⁶ unabhängig voneinander einen Pyrrolidin-1-yl-, Pyrazolidin-1-yl-, lmidazolidin-1-yl, Oxazolidin-3-yl-, Isoxazolidin-2-yl-, Piperidin-1-yl-, Piperazin-1-yl-, Morpholin-4-yl- oder Thiomorpholin-4-yl-Rest, der ein- oder mehrfach durch C₁-C₄-Alkyl, Benzyl, Phenylethyl oder Phenyl substituiert ist und über ein Ringstickstoffatom an das Phthalocyaningerüst gebunden ist,
und
gegebenenfalls die übrigen Reste R¹ bis R¹⁶ Wasserstoff, Halogen, Hydroxysulfonyl oder C₁-C₄-Dialkylsulfamoyl bedeuten,
als Markierungsmittel für Flüssigkeiten, insbesondere für Mineralöle, sowie Mineralöle, enthaltend solche Phthalocyanine.

Aus J. Gen. Chem. USSR, Band 51, Seiten 1405 bis 1411, 1981, ist die Herstellung von Tetrakispiperidinylphthalocyanin bekannt. Weiterhin werden in der WO-A-94/02570 und WO-A-96/10620 Phthalocyanine als Markierungsmittel für Flüssigkeiten, insbesondere Mineralöle, beschrieben.

Es hat sich jedoch gezeigt, daß die dort beschriebenen Markierungsmittel noch Nachteile in ihren anwendungstechnischen Eigenschaften, insbesondere eine ungenügende Löslichkeit und eine ungenügende chemische Stabilität in Lösung aufweisen.

Aufgabe der vorliegenden Erfindung war es daher, geeignete Phthalocyanine bereitzustellen, die ein verbessertes Eigenschaftsprofil besitzen.

Demgemäß wurden die eingangs näher bezeichnete Verwendung von Phthalocyaninen der Formel I gefunden.

Alle in den hier genannten Formeln auftretenden Alkylreste können sowohl geradkettig als auch verzweigt sein.

Halogen bedeutet z.B. Fluor, Chlor, Brom oder Iod.

C₁-C₄-Dialkylsulfamoyl bedeutet z.B. Dimethylsulfamoyl, Diethylsulfamoyl, Dipropylsulfamoyl, Diisopropylsulfamoyl, Dibutylsulfamoyl oder N-Methyl-N-ethylsulfamoyl.

Die heterocyclischen Reste können ein- oder mehrfach, vorzugsweise ein- bis dreifach, insbesondere einfach, durch C₁-C₄-Alkyl, Benzyl, Phenylethyl oder Phenyl substituiert sein.

Geeignete heterocyclische Reste sind z.B. Pyrrolidin-1-yl, 2-oder 3-Methylpyrrolidin-1-yl, 2,4-Dimethyl-3-ethylpyrrolidinyl, Pyrazolidin-1-yl, 2-, 3-, 4- oder 5-Methylpyrazolidin-1-yl, Imidazolidin-1-yl, 2-, 3-, 4- oder 5-Methylimidazolidin-1-yl, Oxazolidin-3-yl, 2-, 4- oder 5-Methyloxazolidin-3-yl, Isoxazolidin-2-yl, 3-, 4- oder 5-Methylisoxazolidin-2-yl, Piperidin-1-yl, 2-, 3-, 4-Methyl-, -Ethyl- oder -Benzylpiperidin-1-yl, 2,6-Dimethylpiperidin-1-yl, Piperazin-1-yl, 4-(C₁-C₄-Alkyl)piperazin-1-yl, wie 4-Methyl- oder 4-Ethyl piperazin-1-yl, Morpholin-4-yl oder Thiomorpholin-4-yl.

Bevorzugte heterocyclische Reste leiten sich von Pyrrolidin, Piperidin, Piperazin oder Morpholin als Grundkörper ab.

Bevorzugt sind Phthalocyanine der Formel I, in der vier der Reste R¹ bis R¹⁶ einen heterocyclischen Rest bedeuten.

Weiterhin bevorzugt sind Phthalocyanine der Formel I, in der vier der Reste R¹ bis R¹⁶ einen heterocyclischen Rest und die übrigen Reste R¹ bis R¹⁶ Wasserstoff bedeuten.

Besonders bevorzugt finden Verwendung Phthalocyanine, die der Formel Ia oder Ib 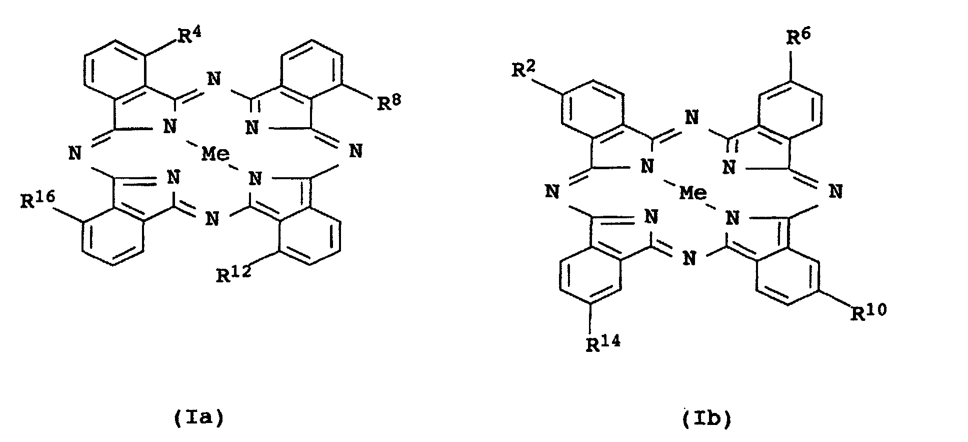 gehorchen,
in der die Reste R⁴, R⁸, R¹² und R¹⁶ sowie R², R⁶, R¹⁰ und R14 jeweils einen heterocyclischen Rest bedeuten und Me jeweils die obengenannte Bedeutung besitzt, sowie deren Stellungsisomere in Bezug auf die Reste R⁴, R⁸, R¹² und R¹⁶ sowie R², R⁶, R¹⁰ und R¹⁴.

Von besonderem Interesse ist die Verwendung von Phthalocyaninen der Formel Ia oder Ib, in der R⁴, R⁸, R¹² und R¹⁶ sowie R², R⁶, R¹⁰ und R¹⁴ jeweils Pyrrolidin-1-yl, Piperidin-1-yl, Piperazin-1-yl oder Morpholin- 4-yl bedeuten, wobei diese Reste ein- bis dreifach, vorzugsweise
einfach, durch C₁-C₄-Alkyl, Benzyl, Phenylethyl oder Phenyl substituiert sein können.

Daneben werden Phthalocyanine der Formel I bevorzugt verwendet, in denen die Substituenten aus einer Kombination der oben aufgeführten bevorzugten Substituenten ausgewählt sind.

Die Phthalocyanine der Formel I können auf an sich bekannte Weise erhalten werden, wie sie beispielsweise in J.Gen. Chem. USSR, Band 51, Seiten 1405 bis 1411, 1981, F.H. Moser, A.L. Thomas "The Phthalocyanines", CRC Press, Boca Rota, Florida, 1983, oder J. Am. Chem. Soc. Band 106, Seiten 7404 bis 7410, 1984, beschrieben ist. So kann man z.B. Phthalodinitrile, die, entsprechend Formel I, geeignete Substituenten tragen, in einem inerten Verdünnungsmittel in Gegenwart einer Base, gegebenenfalls in Gegenwart eines Metallisierungsreagenz zur Umsetzung bringen.

Es ist häufig erforderlich, Flüssigkeiten zu markieren, um in der Folge, z.B. bei ihrer Anwendung, mittels geeigneter Methoden die so markierten Flüssigkeiten wieder zu detektieren.

Beispielsweise kann auf diese Weise Heizöl von Dieselöl unterschieden werden.

Geeignete Lösungsmittel, die erfindungsgemäß mittels der oben näher bezeichneten Verbindungen markiert werden können, sind insbesondere organische Flüssigkeiten, beispielsweise Alkohole, wie Methanol, Ethanol, Propanol, Isopropanol, Butanol, Isobutanol, sec-Butanol, Pentanol, Isopentanol, Neopentanol oder Hexanol, Glykole, wie 1,2-Ethylenglykol, 1,2- oder 1,3-Propylenglykol, 1,2-, 2,3- oder 1,4-Butylenglykol, Di- oder Triethylenglykol oder Di- oder Tripropylenglykol, Ether, wie Methyltertbutylether, 1,2-Ethylenglykolmono- oder -dimethylether, 1,2-Ethylenglykolmono- oder -diethylether, 3-Methoxypropanol, 3-Isopropoxypropanol, Tetrahydrofuran oder Dioxan, Ketone, wie Aceton, Methylethylketon oder Diacetonalkohol, Ester, wie Essigsäuremethylester, Essigsäureethylester, Essigsäurepropylester oder Essigsäurebutylester, aliphatische oder aromatische Kohlenwasserstoffe wie Pentan, Hexan, Heptan, Octan, Isooctan, Petrolether, Toluol, Xylol, Ethylbenzol, Tetralin, Dekalin, Dimethylnaphthalin, Testbenzin, Mineralöle, wie Benzin, Kerosin, Dieselöl oder Heizöl, natürliche Öle, wie Olivenöl, Sojaöl oder Sonnenblumenöl, oder natürliche oder synthetische Motoren-, Hydraulikoder Getriebeöle, z.B. Fahrzeugmotorenöl oder Nähmaschinenöl, oder Bremsflüssigkeiten.

Besonders vorteilhaft verwendet man die obengenannten Verbindungen zum Markieren von Mineralölen, bei denen gleichzeitig eine Kennzeichnung gefordert wird, z.B. aus steuerlichen Gründen. Um die Kosten der Kennzeichnung gering zu halten, strebt man üblicherweise an, für die Färbung möglichst ausgiebige Farbstoffe zu verwenden. Jedoch sind selbst sogenannte farbstarke Farbstoffe in hoher Verdünnung in Mineralölen rein visuell nicht mehr wahrnehmbar.

Bezogen auf das Gewicht der zu markierenden Flüssigkeit wendet man 1 bis 1000 ppb, vorzugsweise 1 bis 500 ppb und insbesondere 100 ppb bis 500 ppb Phthalocyanin I an.

Zum Markieren der Flüssigkeiten, insbesondere von Mineralöl, werden die Phthalocyanine der Formel I im allgemeinen in Form von Lösungen angewandt. Als Lösungsmittel eignen sich vorzugsweise aromatische Kohlenwasserstoffe, wie C₁-C₂₀-alkylsubstituierte aromatische Kohlenwasserstoffe, z.B. Toluol, Xylol oder Shellsol® (Fa. Shell). Um eine zu hohe Viskosität der resultierenden Lösungen zu vermeiden, wählt man im allgemeinen eine Konzentration an Phthalocyanin I von 0,5 bis 60 Gew.-%, bezogen auf die Lösung.

Ein weiterer Gegenstand der vorliegenden Erfindung sind Mineralöle, enthaltend eines- oder mehrere Phthalocyanine der Formel I.

Die Phthalocyanine I weisen in der Regel ihr Absorptionsmaximum im Bereich von 600 bis 1 200 nm auf und/oder fluoreszieren im Bereich von 620 bis 1 200 nm und können so mit geeigneten Instrumenten leicht detektiert werden.

Der Nachweis der Phthalocyanine I kann auf an sich bekannte Weise, z.B. durch Messung des IR-Absorptionsspektrums der zu untersuchenden Flüssigkeiten, erfolgen.

Man kann aber auch die Fluoreszenz der in den Flüssigkeiten enthaltenen Phthalocyanine I anregen, vorteilhaft mit einem Halbleiterlaser oder einer Halbleiterdiode. Besonders günstig ist es, dabei einen Halbleiterlaser oder eine Halbleiterdiode mit einer Wellenlänge der maximalen Emission im Spektralbereich von λₘₐₓ -100 nm bis λₘₐₓ +20 nm anzuwenden. λₘₐₓ bedeutet dabei die Wellenlänge des Absorptionsmaximums des Markierstoffs. Die Wellenlänge der maximalen Emission liegt dabei im Bereich von 620 bis 1 200 nm.

Das so erzeugte Fluoreszenzlicht wird vorteilhaft mit einem Halbleiterdetektor, insbesondere mit einer Silicium-Photodiode oder einer Germanium-Photodiode, detektiert.

Besonders vorteilhaft gelingt der Nachweis, wenn sich vor dem Detektor noch ein Interferenzfilter und/oder ein Kantenfilter (mit einer kurzwelligen Transmissionskante im Bereich von λₘₐₓ bis ₘₐₓ +80 nm) und/oder ein Polarisator befindet.

Mittels der obengenannten Verbindungen gelingt es sehr einfach, markierte Flüssigkeiten nachzuweisen, selbst wenn die Phthalocyanine I nur in einer Konzentration von ungefähr 1 ppm (Nachweis durch Absorption) oder ungefähr 5 ppb (Nachweis durch Fluoreszenz) vorliegen.

Die Phthalocyanine der Formel I zeichnen sich durch eine hohe Löslichkeit in der zu markierenden Flüssigkeiten aus. Außerdem verfügen sie über eine hohe chemische Stabilität in Lösung.

Die folgenden Beispiele sollen die Erfindung näher erläutern.

### A) Herstellung

### Beispiel 1

In 1 l n-Butanol wurden 56,3 g (0,325 mol) 30 gew.-%iger methanolische Natriummethanolatlösung gelöst und das überschüssige Methanol solange abdestilliert, bis ein konstanter Siedepunkt von 117°C erreicht wurde. Anschließend wurden 112,5 g 3-(3'-Methylpiperidin-1-yl)phthalodinitril eingetragen und 6 h unter Rückfluß gerührt. Dann wurde auf 1,5 l Methanol gegeben, 1 h nachgerührt und abgesaugt. Der Rückstand wurde sukzessive mit Methanol, Wasser und Aceton gewaschen und dann an der Luft getrocknet. Man erhielt 101,4 g Phthalocyanin der Formel 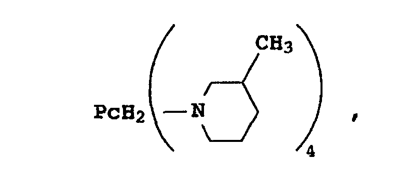 worin PcH₂ den vierwertigen Rest von Phthalocyanin bedeutet, dessen Zentraleinheit zweimal Wasserstoff ist.

In analoger Weise werden die in der folgenden Tabelle 1 aufgeführten Phthalocyanine erhalten.

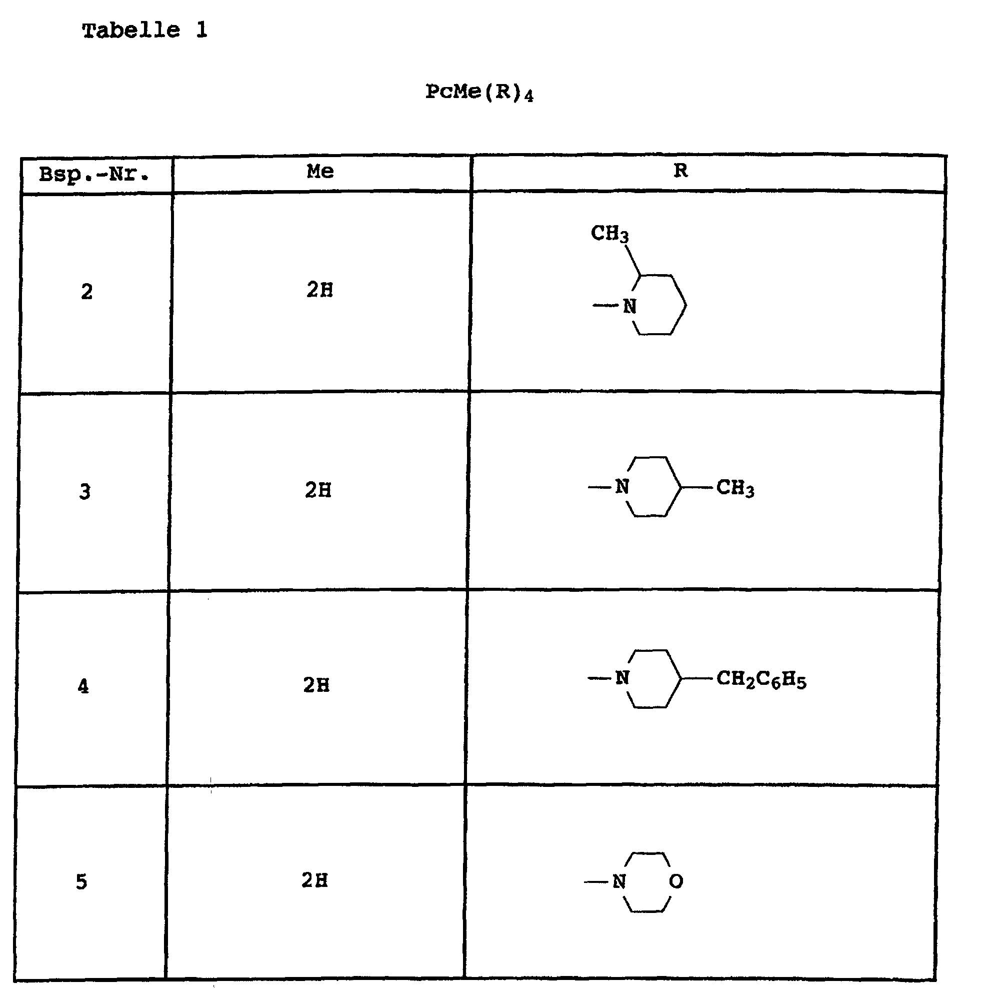

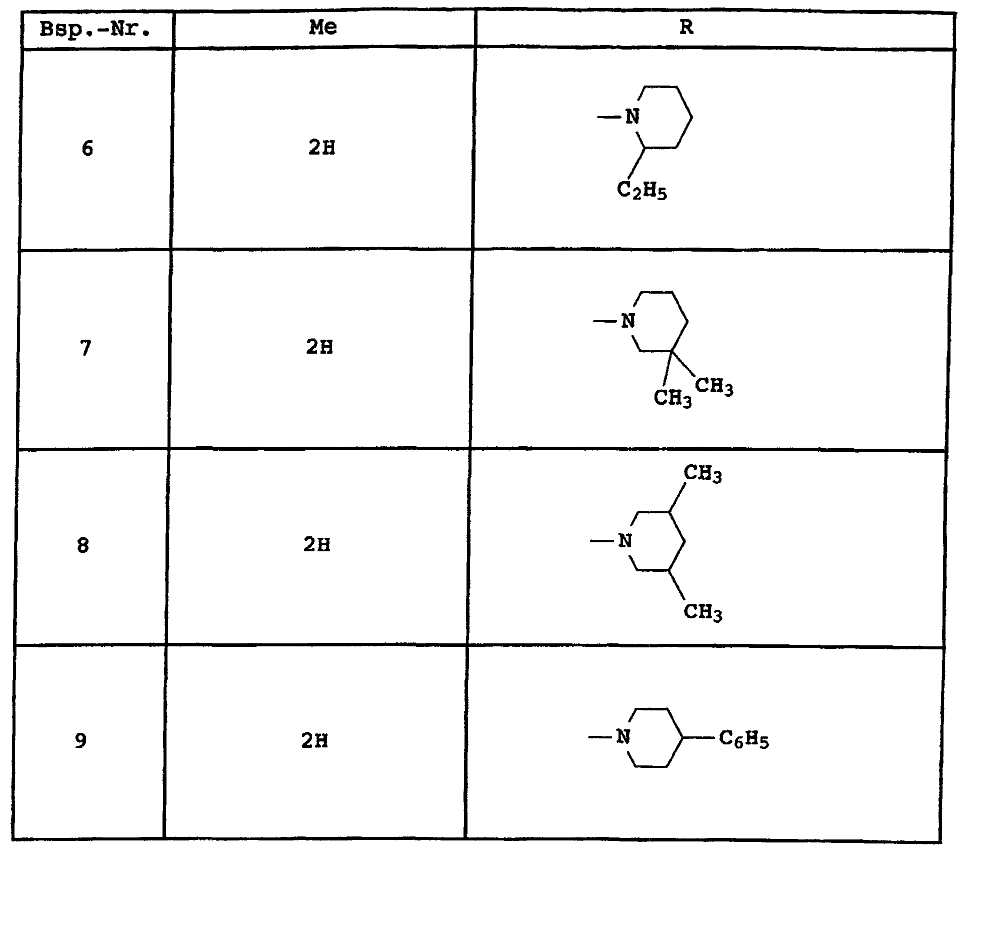

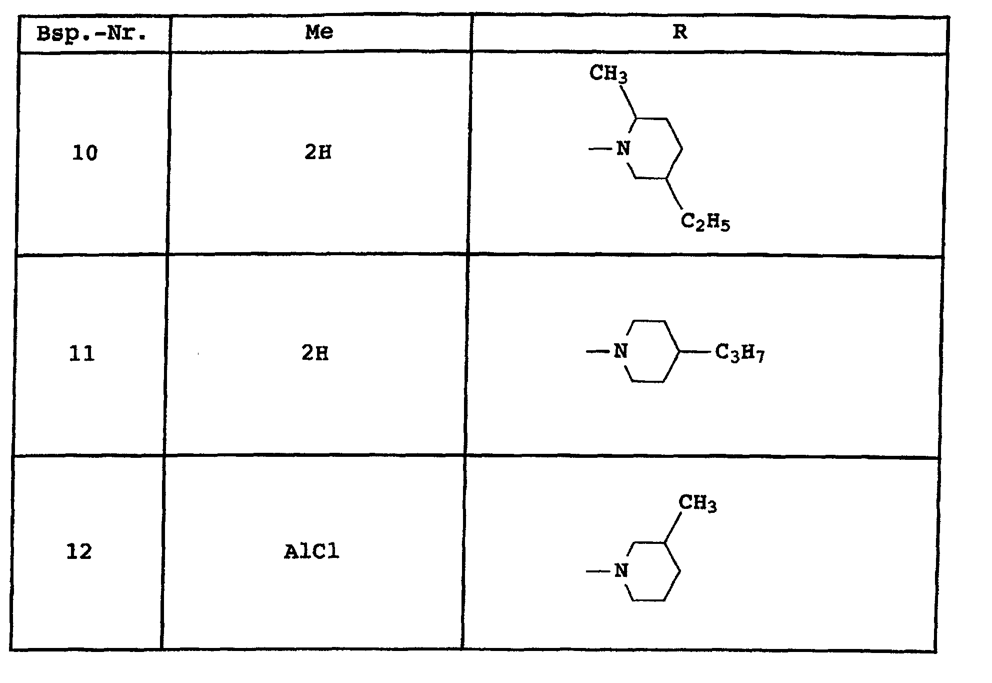

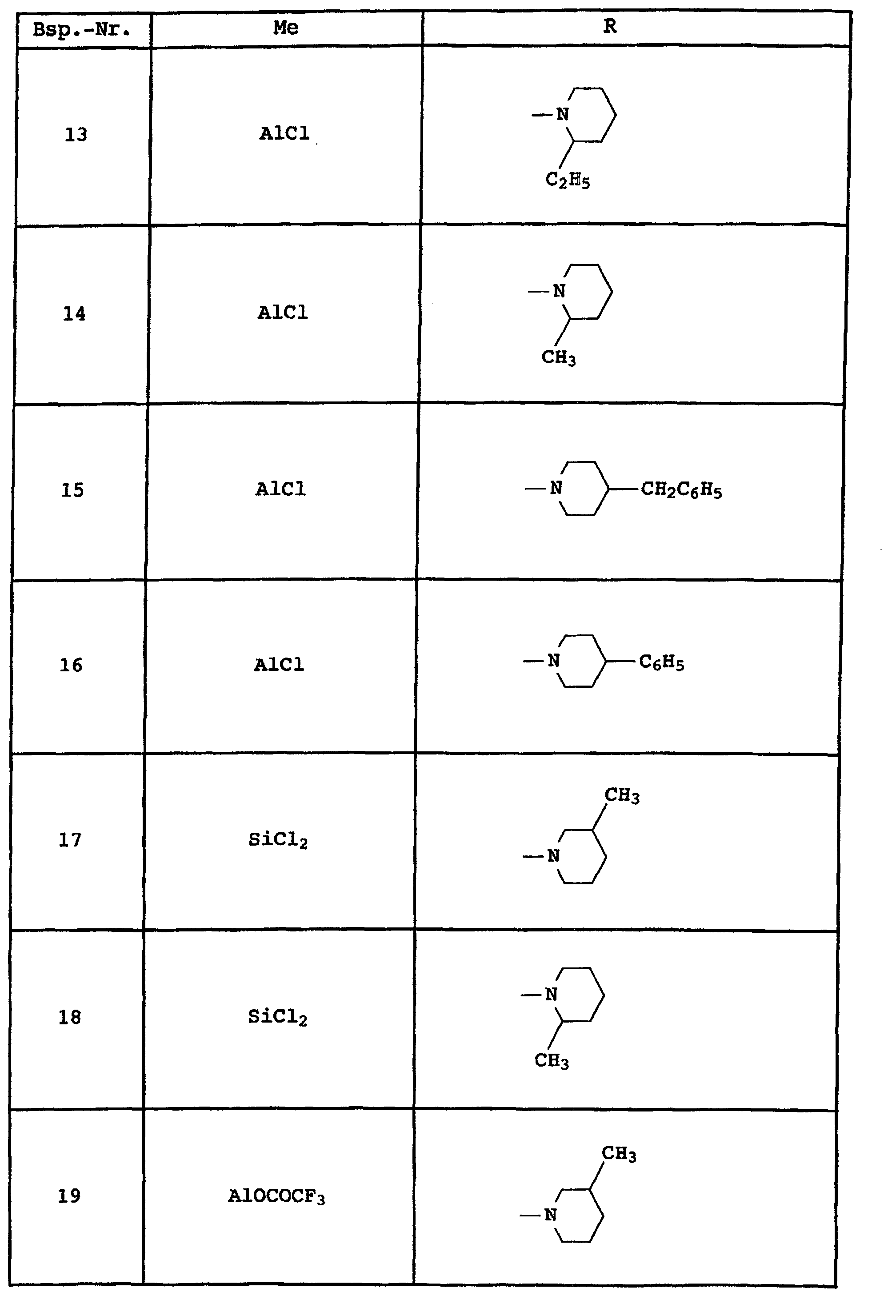

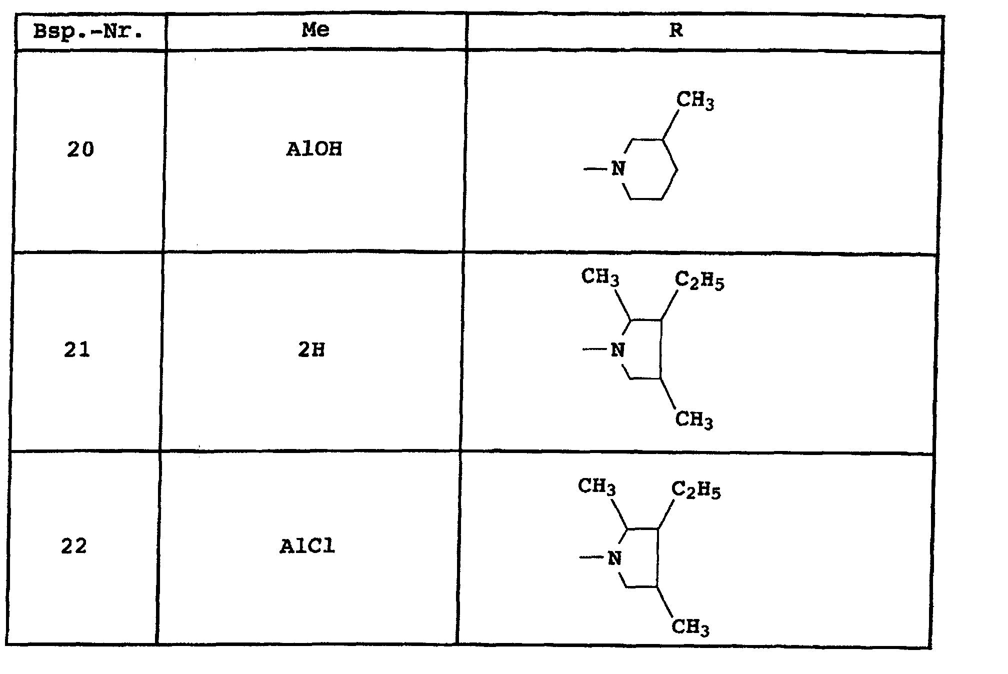

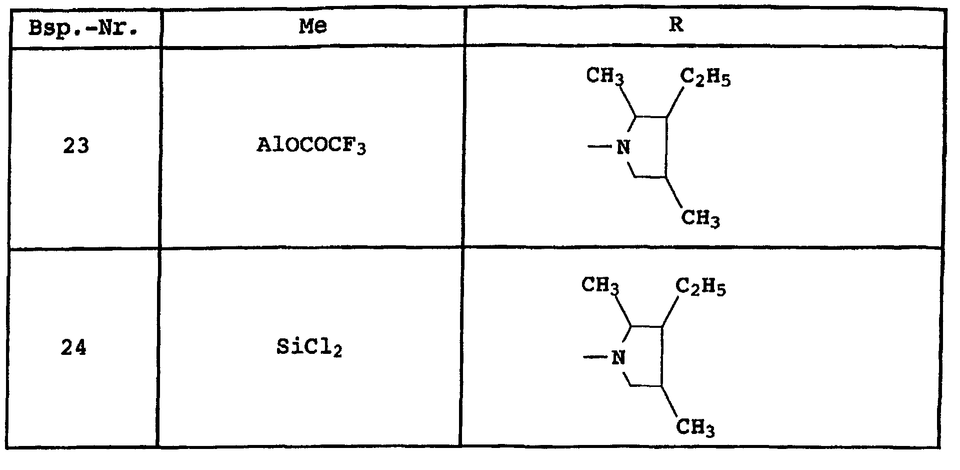

### B) Anwendung

### I. Nachweis durch Absorption im IR-Bereich

Es wurde soviel Farbstoff der Formel 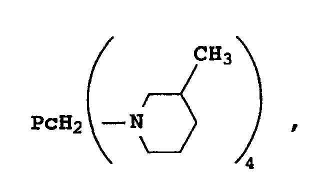 in einer der in Tabelle 2 genannten Flüssigkeiten gelöst, daß eine Lösung mit einem Gehalt an Farbstoff von 10 ppm erhalten wurde. Die Absorption dieser Lösungen im IR-Bereich wurde jeweils mittels eines handelsüblichen Spektrometers (1 cm-Küvette) vermessen.

**Tabelle 2**

| Farbstoffgehalt [ppm] | Absorption | Absorptionsmaximum [nm] |
|---|---|---|
| Dieselkraftstoff | 1,10 | 762 |
| Bleifreies Benzin | 1,10 | 760 |
| Ethanol | 1,05 | 771 |
| Toluol | 1,15 | 770 |

Zur Messung der Lagerstabilität des Farbstoffs wurden die Proben mehrere Wochen bei Raumtemperatur (RT) und bei 50°C gelagert und die Absorption mit einem handelsüblichen Spektrometer vermessen. Im einzelnen wurden folgende Ergebnisse erhalten.

**Tabelle 3**

| Versuchsdauer (Temp.) | Ethanol | Toluol | Dieselkraftstoff | Bleifreies Benzin |
|---|---|---|---|---|
| 0 Wochen (RT) | 1,0535 | 1,1486 | 1,0964 | 1,0993 |
| 1 Woche (RT) | 1,0601 | 1,152 | 1,0977 | 1,0937 |
| 2 Wochen (RT) | 1,0479 | 1,1484 | 1,097 | 1,1014 |
| 4 Wochen (RT) | 1,0467 | 1,1517 | 1,097 | 1,098 |
| 8 Wochen (RT) | 1,0181 | 1,1443 | 1,078 | 1,0869 |
| 1. Woche (50°C) | 1,0467 | 1,1421 | 1,016 | 1,0926 |
| 2 Wochen (50°C) | 1,0521 | 1,1538 | 1,0917 | 1,0989 |
| 4 Wochen (50°C) | 1,043 | 1,1438 | 1,0937 | 1,0864 |
| 8 Wochen (50°C) | 1,0467 | 1,1445 | 1,0455 | 1,0617 |

### II. Nachweis durch Fluoreszenz im NIR-Bereich

Zur Anregung der Marker-Fluoreszenz wird die Emission eines kommerziellen Halbleiterdiodenlasers benutzt. Der parallele Laserstrahl wird auf die in einer 1-cm-Küvette befindliche Probe gestrahlt. Zur Verdoppelung der Anregungsintensität wird der transmittierte Lichtstrahl durch einen Spiegel reflektiert und nochmals durch die Probe gestrahlt.

Das Fluoreszenzlicht wird mittels optischer Elemente (Linsensystem) auf den Detektor, eine Silicium-Photodiode, abgebildet. Das rückseitig abgestrahlte Licht wird von einem Hohlspiegel ebenfalls auf die Silicium-Photodiode geworfen.

Zur Abtrennung des Störlichts (gestreutes Anregungslicht) vom Fluoreszenzlicht werden Kanten- und/oder Interferenzfilter und/oder ein Polarisator (NIR-Polarisationsfolie) benutzt.

Die Optimierung des Polarisators wird dabei so gewählt, daß die Richtung der maximalen Transmission senkrecht zur Polarisationsebene des Anregungslichts steht.

Es wurde soviel Farbstoff der Formel 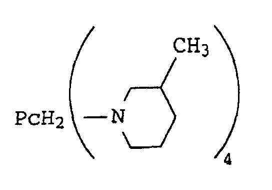 in Dieselkraftstoff gelöst, daß eine Lösung mit einem Gehalt an Farbstoff von 250 ppb erhalten wurde.

Diese Lösung wurde gemäß der allgemeinen Vorschrift II unter Anwendung der folgenden apparativen Größen vermessen.

Anregung: Halbleiterdiodenlaser mit der Laserwellenlänge 789 nm; CW-Leistung 2 mW (Modulation: 1,9 kHz)

Filter: Langpaß-Interferenzfilter 805 nm.

Photodetektor: Silicium-PIN-Diode mit einer Fläche von 1 cm². Der Photostrom wurde von einem Lockin-Verstärker detektiert. Wesentlicher Gesichtspunkt dieser Messungen war die Lagerstabilität des Farbstoffs bei Raumtemperatur. Folgende in Tabelle 3 aufgeführten Meßwerte wurden erhalten.

**Tabelle 3**

| Zeit [Wochen] | Extinktion bei λₘₐₓ [nm] | Fluoreszenzsignal (in Skalenteilen) |
|---|---|---|
| 0 | 789 | 1,96 |
| 1 | 789 | 1,98 |
| 2 | 789 | 2,04 |
| 3 | 789 | 1,90 |
| 4 | 789 | 1,95 |

## Patentansprüche

1. Verwendung von Phthalocyaninen der Formel I 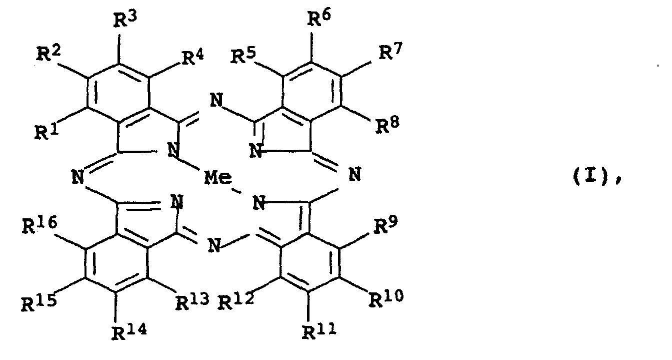 in der
Me zweimal Wasserstoff, zweimal Lithium, Magnesium, Zink, Kupfer, Nickel, VO, TiO, AlCl, AlOH, AlOCOCH₃, AlOCOCF₃, SiCl₂ oder Si(OH)₂,
mindestens vier der Reste R¹ bis R¹⁶ unabhängig voneinander einen Pyrrolidin-1-yl-, Pyrazolidin-1-yl-, Imidazolidin-1-yl, Oxazolidin-3-yl-, Isoxazolidin-2-yl-, Piperidin-1-yl-, Piperazin-1-yl-, Morpholin-4-yl- oder Thiomorpholin-4-yl-Rest, der ein- oder mehrfach durch C₁-C₄-Alkyl, Benzyl, Phenylethyl oder Phenyl substituiert ist und über ein Ringstickstoffatom an das Phthalocyaningerüst gebunden ist,
und
gegebenenfalls die übrigen Reste R¹ bis R¹⁶ Wasserstoff, Halogen, Hydroxysulfonyl oder C₁-C₄-Dialkylsulfamoyl bedeuten,
als Markierungsmittel für Flüssigkeiten.

2. Verwendung von Phthalocyaninen der Formel I gemäß Anspruch 1 als Markierungsmittel für Mineralöle.

3. Mineralöle, enthaltend eine oder mehrere Phthalocyanine der Formel I gemäß Anspruch 1.

## Claims

1. Use of phthalocyanines of the formula I 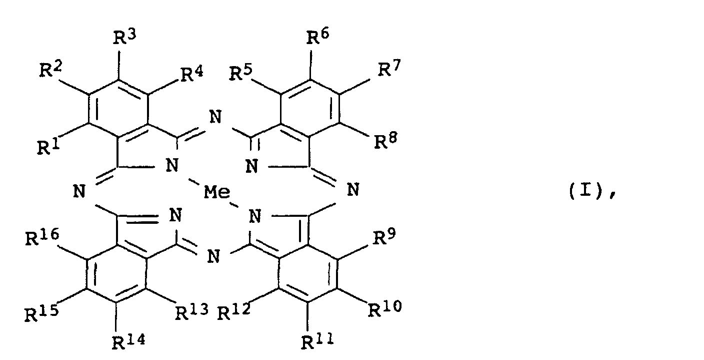 where
Me is twice hydrogen, twice lithium, magnesium, zinc, copper, nickel, VO, TiO, AlCl, AlOH, AlOCOCH₃, AlOCOCF₃, SiCl₂ or Si(OH)₂,
at least four of the radicals R¹ to R¹⁶ are each independently of the others pyrrolidin-1-yl, pyrazolidin-1-yl, imidazolidin-1-yl, oxazolidin-3-yl, isoxazolidin-2-yl, piperidin-1-yl, piperazin-1-yl, morpholin-4-yl or thiomorpholin-4-yl, which radicals can be monosubstituted or polysubstituted by C₁-C₄-alkyl, benzyl, phenylethyl or phenyl, and which are bonded to the phthalocyanine structure via a ring nitrogen atom,
and
any remaining radicals R¹ to R¹⁶ are each hydrogen, halogen, hydroxysulfonyl or C₁-C₄-dialkylsulfamoyl,
as markers for liquids.

2. Use of phthalocyanines of the formula I as set forth in claim 1 as markers for mineral oils.

3. Mineral oils comprising one or more phthalocyanines of the formula I as set forth in claim 1.

## Revendications

1. Utilisation de phtalocyanines de formule I 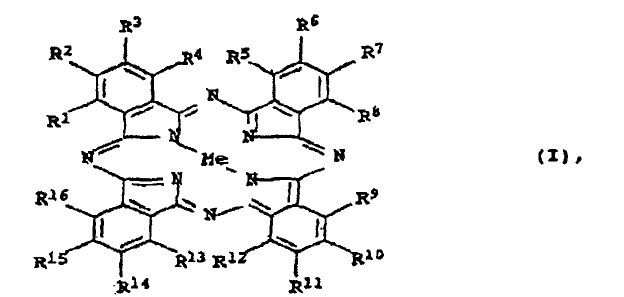 dans laquelle
Me signifie deux fois un atome d'hydrogène, deux fois un atome de lithium, de magnésium, de zinc, de cuivre, de nickel, VO, TiO, AlCl, AlOH, AlOCOCH₃, AlOCOCF₃, SiCl₂ ou Si(OH)₂
au moins quatre des radicaux R¹ à R¹⁶ signifient, indépendamment l'un de l'autre, un radical pyrrolidin-1-yle, pyrazolidin-1-yle, imidazolidin-1-yle, oxazolidin-3-yle, isoxazolidin-2-yle, pipéridin-1-yle, pipérazin-1-yle, morpholin-4-yle ou thiomorpholin-4-yle, qui est monosubstitué ou polysubstitué par un groupe alkyle en C₁ à C₄, benzyle, phényléthyle ou phényle et qui est lié à la structure phtalocyanine via un atome d'azote du cycle
et
le cas échéant les autres radicaux R¹ à R¹⁶ signifient un atome d'hydrogène, d'halogène, un groupe hydroxysulfonyle ou (dialkyle en C₁ à C₄)sulfamoyle,
comme agent de marquage pour des liquides.

2. Utilisation de phtalocyanines de formule I selon la revendication 1 comme agent de marquage pour des huiles minérales.

3. Huiles minérales contenant une ou plusieurs phtalocyanines de formule I selon la revendication 1.
